# EUROPEAN PATENT SPECIFICATION

(11) **EP 2 732 053 B1**
(45) Date of publication and mention of the grant of the patent: **25.10.2017**
(21) Application number: 12811977.3
(22) Date of filing: 13.07.2012
(51) Int. Cl.: C12Q 1/68, C12M 1/00

(54) **SYSTEMS, APPARATUS AND METHODS FOR BIOCHEMICAL ANALYSIS**
SYSTEME, VORRICHTUNGEN UND VERFAHREN FÜR BIOCHEMISCHE ANALYSEN
SYSTÈMES, APPAREILS ET PROCÉDÉS D'ANALYSE BIOCHIMIQUE À HAUT DÉBIT

(30) Priority: 14.07.2011 US 201161507966 P
(43) Date of publication of application: 21.05.2014
(73) Proprietor: Progenity, Inc., San Diego CA 92122 (US)
(72) Inventor: AHN, Keunho, San Diego, California 92122 (US); LAI, Benjamin, San Diego, California 92124 (US); PYFER, Andree, J., Encinitas, California 92024 (US); ZHANG, Yi, San Diego, California 92122 (US); ZHANG, Haichuan, San Diego, California 92126 (US)
(74) Representative: Boult Wade Tennant
(86) International application number: PCT/US2012/046776
(87) International publication number: WO 2013/010134

(56) References cited:
- WO-A1-2005/075081
- WO-A1-2009/021215
- WO-A1-2010/115154
- WO-A2-2006/127191
- WO-A2-2009/108260
- US-A1- 2004 209 288
- US-A1- 2009 042 737
- US-B2- 7 745 221

## Description

### FIELD OF THE INVENTION

Systems, apparatus and methods are provided for biochemical analysis of a sample (e.g., a cell or nucleic acids). Samples are analyzed for molecular information and preferably remain accessible for subsequent analysis or testing. The systems, apparatus and methods provided are useful for performing quantitative and highly parallel biochemical reactions on biological samples in a high-throughput manner.

### BACKGROUND OF THE INVENTION

Reactions that are conducted in solution such as, for example, biochemical reactions, are frequently carried out within a chamber or other container. Such chambers, or reaction vessels, are commonly made of glass or plastic and include, for example, test tubes, microcentrifuge tubes, capillary tubes and microtiter plates. Reaction chambers currently in use are not amenable for use with volumes below one microliter, due to problems such as large head volumes in the reaction chamber leading to evaporative losses of the reaction solution, and difficultly in adding and removing reaction mixtures from the reaction chamber. Accordingly, available reaction chambers do not provide means for readily recovering reaction mixtures for subsequent testing or analysis.

Many types of biochemical reactions, for example, nucleic acid amplification, require temperature cycling. Many reaction chamber materials are poor thermal conductors, thus there are time lags associated with changing the temperature of the reaction vessel and equilibration of a temperature change throughout the sample volume. Such lags in temperature change and temperature equilibration lead to longer cycle times, non-uniform reaction conditions within a single reaction, and lack of reproducibility among multiple reactions, both simultaneous and sequential.

It is often necessary to carry out a series of experiments on a large number of identical samples. Usually this set of samples must be serially duplicated, either manually or by means of robotic liquid delivery systems. These processes can be slow, as they depend on the total number of samples to be duplicated and, if applicable, the speed of the robot. Additionally, is may be necessary to carry out multiple biochemical reactions on the same sample. Current systems do not permit serial reactions to be performed on samples.

Real-time polymerise chain reactions (qPCR) are a technique used to quantitatively measure DNA and RNA extracted from biological samples (e.g., cells or plasma). Most qPCR reactions are done in bulk reactions using the pooled genomic equivalent of 10,000 to 100,000 cells. Increasingly, researchers are interested in measuring the genetic contents of biological samples, including, for example, individual cells or free nucleic acids in plasma, but this effort is impeded by the high cost of reagents and the labor intensive manual approaches available today. Even state of the art robotics and 1536 micro-well plates use volumes in the range of 1-10 µL per well still become costly beyond a few hundred wells. In cases where rare events (e.g., rare alleles) that may occur in a small percentage of the genetic material of interest, it may be desirable to examine thousands of aliquots of the biological sample one-by-one. Current technologies cannot achieve this level of throughput without significant costs in time and money. Further, current technologies are end-point systems and do not readily allow for recovery of genetic material following biochemical reactions for subsequent testing or analysis, such as, for example, sequencing.

Thus, there is a need for systems, apparatuses, and methods suitable for performing biochemical reactions on microvolume biological samples. There is also a need for improved methods of recovering such reacted samples for subsequent analysis or testing.

### SUMMARY OF THE INVENTION

The present invention provides a system for performing a biochemical reaction on a sample (e.g., cell or nucleic acids). In particular, there is provided an integrated system for performing a biochemical reaction on a sample as set out in claim 1. Further optional features of the system are set out in dependent claims 2-11.

### BRIEF DESCRIPTION OF THE FIGURES

FIG. 1 shows an embodiment of an integrated system of the present disclosure.
FIG. 2 shows a block diagram of an embodiment of an integrated system of the present disclosure.
Fig. 3A shows an embodiment of a device useful for the systems, apparatus and methods of the present disclosure.
FIG. 3B shows an illustration of a plurality of reaction volumes on a device of the present disclosure.
FIGS. 4A-B show an embodiment of a device useful for the system, apparatus and methods of the present disclosure.
FIG. 5 shows an embodiment of a device useful for the system, apparatus and methods of the present disclosure.
FIG. 6A shows an embodiment of an apparatus of the present disclosure for containing a slide.
FIG. 6B shows an embodiment of an apparatus of the present disclosure containing a slide.
FIG. 7 shows an embodiment of an apparatus of the present disclosure for containing a multi-well plate.
FIG. 8 illustrates contact angles of a reaction volume on a planar surfaces.

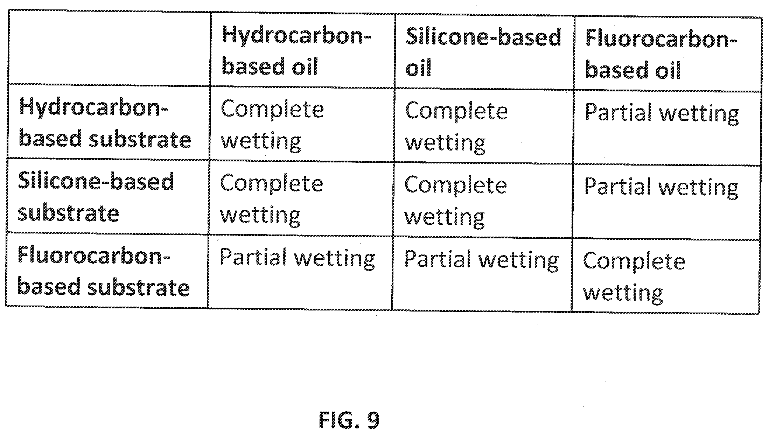

FIG. 9 shows an embodiment of a volatile oil strategy of the present disclosure.
FIGS. 10A-B show an embodiment of a dispensing module of the present disclosure.
FIG. 11 shows an embodiment of a recovery module of the present disclosure.
FIG. 12 shows an embodiment of a recovery module of the present disclosure.

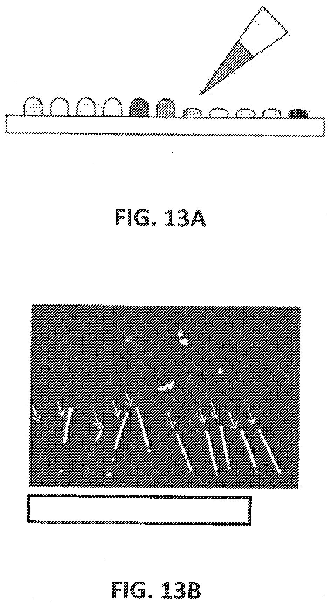

FIGS. 13A-B show embodiments of recovery modules of the present disclosure.
FIG. 14 shows an embodiment of a dual sided thermal module of the present disclosure.
FIGS. 15A-B set forth data showing systems and methods of embodiments of the present disclosure are useful for performing genetic analysis on samples containing nucleic acids.
FIG. 16 sets forth data showing systems and methods of embodiments of the present disclosure are useful for performing serial biochemical reaction on cells.
FIG. 17 sets forth data showing systems and methods of embodiments of the present disclosure are useful for performing serial biochemical reaction on cells and subsequent genomic analysis.
FIG. 18 sets forth recovery results for an embodiment of a recovery module of the present disclosure.

### DESCRIPTION OF THE INVENTION

Before the present systems, apparatus and methods are described, it is to be understood that the invention is not limited to the particular methodologies, protocols, assays, and reagents described, as these may vary. It is also to be understood that the terminology used herein is intended to describe particular embodiments of the present invention, and is in no way intended to limit the scope of the present invention as set forth in the appended claims.

It must be noted that as used herein and in the appended claims, the singular forms "a," "an," and "the" include plural references unless context clearly dictates otherwise. Thus, for example, a reference to "a genetic condition" may include a plurality of such conditions; a reference to a "fetal genetic variation" may be a reference to one or more fetal genetic variations, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present disclosure, the preferred methods, systems, and materials are now described.

### Integrated Systems

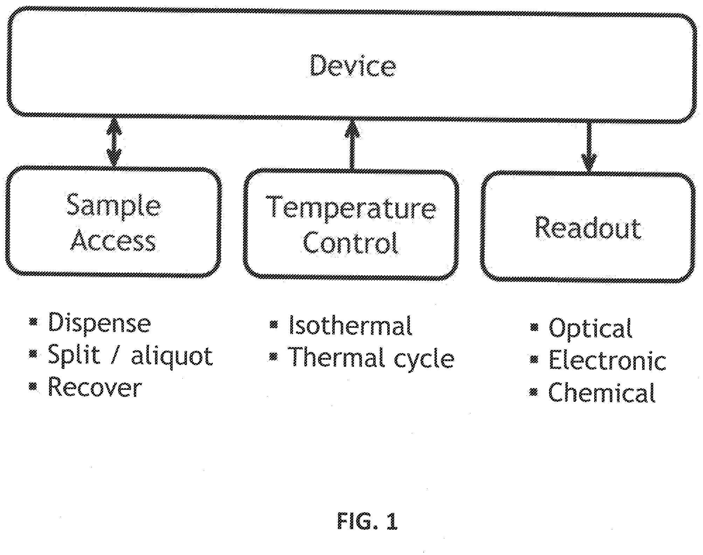

The disclosures provide integrated systems, apparatus and methods for biochemical analysis of a sample (e.g., a cell or free nucleic acids). Samples are analyzed for genetic information and preferably remain accessible for subsequent analysis or testing. The methods and systems provided are useful for performing quantitative and highly parallel biochemical reactions on biological samples in a high throughput manner. In some embodiments, described more fully herein, one or more oils, either non-volatile oil or volatile oils, or a combination of multiple oils, provide an oil strategy to facilitate access to a sample following a biochemical reaction. As shown in FIG 1, the integrated systems of the disclosure comprise a device configured to receive a plurality of discrete reaction volumes and provide access to said reaction volumes, the device can be operably coupled with various modules, including, for example, sample access modules, temperature modules, and readout modules. Sample access modules useful in the systems and methods of the present disclosure provide for dispensing, splitting, aliquoting, and/or recovering a sample to or from a device. Temperature control modules useful in the systems and methods of the present disclosure provide for isothermal or thermal cycling conditions for performing biochemical reactions on discrete reaction volumes comprising a sample and a reagent. Readout modules useful in the systems and methods of the present disclosure provide for detecting an optical, electrical, or chemical signal from said discrete reaction volumes.

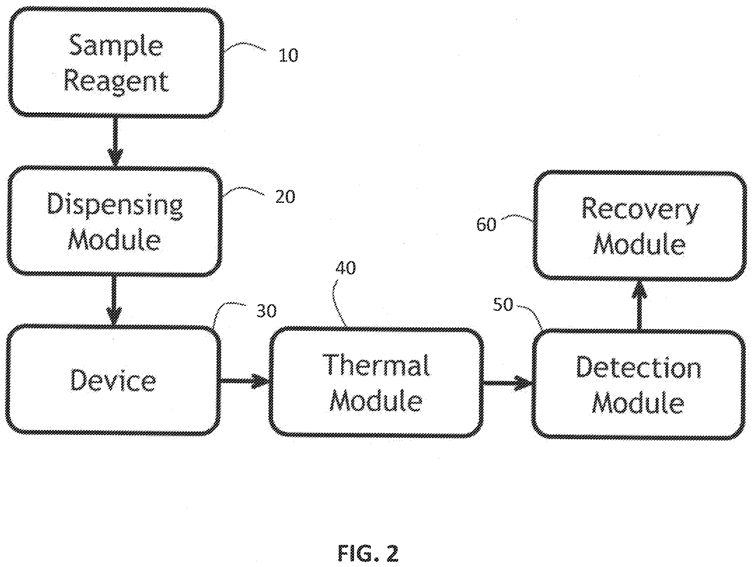

FIG. 2 shows an embodiment of an integrated system of the present disclosure. The system comprises a sample 10 containing nucleic acids and reagents for performing one or more biochemical reactions. A dispensing module 20 comprises a dispenser for dispensing the sample and reagent into or onto a device 30. The device 30 comprises a planar surface or an array of wells configured to receive a plurality of small reaction volumes, such as, for example, nanoliter reaction volumes. The device 30 is configured to be removably and operably coupled to the dispensing module 20, the thermal module 40, the detection module 50, and/or the recovery module 60. The thermal module 40 comprises heating and cooling elements capable of providing isothermal temperature control or cyclic temperature control to facilitate biochemical reactions. The detection module 50 comprises detectors capable of detecting an optical, electrical, or chemical signal from the discrete reaction volumes. The recovery module 60 comprises a recovery apparatus for recovering the material produced by the biochemical reactions.

### Dispensing Module

The present disclosure incorporates the use of methods and devices for dispensing portions of a sample onto or into a device 30 to form a plurality of discrete reaction volumes upon which biochemical reactions, such as, for example, PCR amplification can be performed. The methods and devices used to dispense the sample and any test reagents onto or into the device are not critical to present disclosure, and one of skill in the art will appreciate that many methods and devices may be used to generate such discrete reaction volumes and dispense such samples and reagents. Accordingly, the present disclosure is not intended to be limited to a particular method or device for generating discrete reaction volumes or conducting biochemical reactions on or in a device.

The dispensing module 20 may comprise any one of a number of commercially available reagent dispensers, piezoelectric dispensers, solenoid valve dispensers, and the like. Those of skill in the art will appreciate that other types of dispensers and valve actuation devices exist and may be used efficaciously. Examples of dispensers that may be used in the present disclosure include, for example, air brush dispensers, piezoelectric dispensers, fluid impulse dispensers, heat actuated dispensers, and the like. Suitable dispensers for use in the systems of the present disclosure are described in U.S. Patent Application Pub. No. 2010/0273680 and U.S. Patent No. 6,576,295.

In certain embodiments, a positive displacement syringe pump is hydraulically coupled to the dispenser. Alternatively, the pump may be any one of several varieties of commercially available pumping devices for metering precise quantities of liquid. A wide variety of other direct current fluid sources may be used, however. These may include, without limitation, rotary pumps, peristaltic pumps, squash-plate pumps, and the like, or an electronically regulated fluid current source.

In certain aspects, the sample and/or reagent is dispensed in a volume between about 1 nL to about 500 nL. Discrete reaction volumes generated using the dispensing module 20 described herein may be formed with dispensed sample and/or reagent volumes as small as about 1 nL, 5 nL, 10, nL, 25 nL, or 50 nL. Typically, reaction volumes may range from about 1 nL to about 250 nL, although particular reaction volumes range from about 10 nL to about 250 nL; more particular reaction volumes range from between about 50 nL to about 250 nL. This small reaction volume allows multiple discrete reaction volumes to be applied to a single device 30, such as, for example, a microscope slide, so that between about 1-5, 5-10, 10-100, 500-1000 or more, discrete samples, including a combination of control and test samples, may be formed on or in the device 30. The small sample volumes also significantly reduces the amount of test reagent that is necessary for the biochemical reaction, thereby further decreasing the overall cost of conducting the biochemical reaction while at the same time increasing the fidelity of the results of the biochemical reaction because the test and control samples can be processed under identical conditions. As compared to the volume of a prior art bulk reaction, the preferred reaction volumes of the disclosure is from about 1:20 to 1:100,000 times smaller. Further, since reaction volumes contained in or on a device of the present disclosure remain accessible, multiple biochemical reactions may be carried out with the subsequent dispensing of additional sample or reagent(s).

Several suitable syringe pumps are commercially available to one of skill in the art, For example, the Biodot CV1000 Syringe Pump Dispenser, available from Biodot, Inc. (Irvine, Calif) incorporates an electronically controlled stepper motor for providing precision liquid handling using a variety of syringe sizes. Such syringe pumps may have anywhere from 3,000-24,000 steps, although higher resolution pumps having 48,000-768,000 steps may be used in the integrated systems of the present disclosure. Higher resolution pumps, such as, for example, piezoelectric pumps may also be used to provide even finer resolutions, if desired. Multiple syringe pumps may be employed in parallel, for example, to deliver varying concentrations of reagents and/or other liquids to the dispenser or to alternate dispensing operations between two or more reagents as may be desired when conducting the biochemical reactions contemplated by the present disclosure.

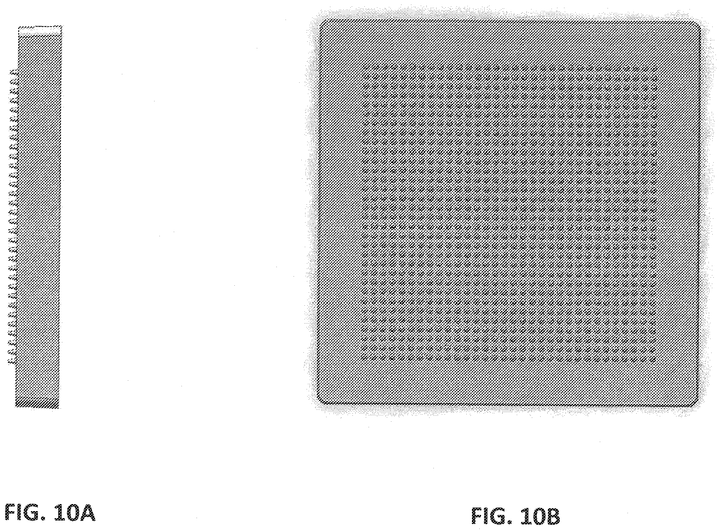

Pin tools may be used to dispense sample and reagent(s) in the systems of the present disclosure. Pin tools refer to devices that are capable of transferring substantially all or a portion of fluid from a fluid source to a fluid destination without drawing the aliquots from the fluid source under an applied pressure. An exemplary pin tool useful in the methods and systems of the present disclosure is shown in FIGS. 10A and 10B. For example, fluid aliquots typically adhere to a pin such that the pin can transfer the aliquots to the destination, e.g., the wells of a multi-well plate or surface of a plastic or glass slide. In particular embodiments, a fluid transfer achieved with a pintool having multiple pins. Typically, the pins of a pintool have a footprint that corresponds to the wells of a multi-well container, such as a standard microtiter plate, so that the pins can access the wells to deposit fluid volumes into or withdraw fluid volumes from the wells substantially simultaneously. More specifically, pintools can have, e.g., 6, 12, 24, 48, 96, 192, 384, 768, 1536, 3456, 9600, or more pins. Pin tools can accurately transfer between 2 and 200 nL of sample and/or reagents to a device used in the methods and systems of the present disclosure. (See, e.g., Cleveland et al. (2005) Assay Drug Dev Technol. 3:213-25.) Pins may be coated with hydrophilic materials to affect fluid transfer volume. Accordingly, any pin tool known to one of skill in the art maybe used in the systems and methods of the present disclosure.

### Devices

The devices used in the methods and systems of the present disclosure are configured to receive a plurality of discrete reaction volumes. In some embodiments, the discrete reaction volumes remain accessible while in or on the device. For example, following a biochemical reaction on a plurality of discrete reaction volumes in a micro-well plate, the discrete reaction volumes remain accessible for recovery or for addition of reagent for performing another biochemical reaction.

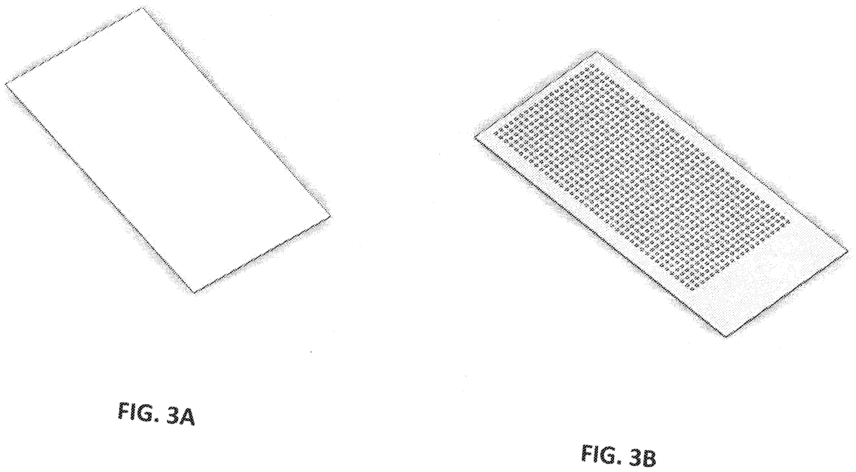

Devices useful in the methods and systems of the present disclosure include planar surfaces, such as, for example, glass or plastic (e.g., polymer) slides. FIGS. 3A and 3B show an exemplary device useful in the methods and systems of the present disclosure. In some embodiments the device used in the system is a planar surface comprising glass or polymer material. In one aspect, the glass is soda-lime glass, borosilicate glass, aluminosilicate glass, lead glass, 96% silica glass, fused silica glass, or quartz. In other aspects, the polymer is PMMA, polystyrene, polycarbonate, polypropylene, polyethylene, high density polyethylene, COC, or COP.

As shown in FIG. 3B, nanoliter samples can be dispensed onto the surface of the slide to form a plurality of discrete volume reactions. For example, a 20 x 40 array of reaction volumes (i.e., 800 reaction volumes) can be dispensed on a planar surface for subsequent testing. Accordingly, the number of discrete volume reactions dispensed onto the surface of the slide may be about 10 to 5000 volume reactions, about 500 to 5000 volume reactions, about 800 to 5000 volume reactions, or about 1000 to 5000 volume reactions. In certain embodiments, the number of discrete volume reactions dispensed onto the surface of the slide is 800, 1152, or 4608. In some embodiments, the discrete volume reactions dispensed onto the surface of the slide are about 1-500 nL, about 5-100 nL, about 5-50 nL, about 5-30 nL, or about 5-10 nL. In specific embodiments, the discrete volume reactions dispensed onto the surface of the slide are about 30 nL. In some embodiments, oligonucleotides (e.g., primers) are lyophilized onto the planar surface prior to the addition of the plurality of discrete reaction volumes.

In some embodiments, the surface of the slide is homogenous. In other embodiments, the surface of the slide is pretreated or coated with a hydrophobic or hydrophilic material. In one aspect, the hydrophobic material is PDMS. In other aspects, the hydrophilic material is PDMS with subsequent oxygen or air plasma treatment, plasma treatment with a mask (i.e., selective treatment or patterning), or silanization. Once the sample and reagent(s) have been dispensed onto the planar surface (e.g., slide surface), oil or any other suitable encapsulation media is applied to isolate the discrete reaction volumes arrayed on the planar surface (See, e.g., FIG 3B).

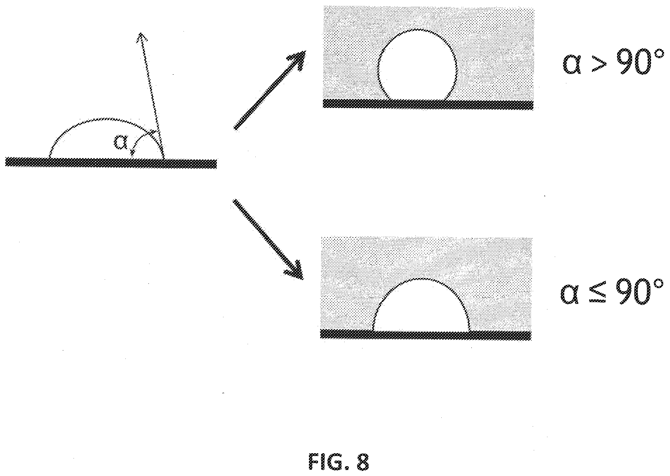

The contact angle and wetting between the reaction volume and planar surface are selected to prevent movement of the discrete reaction volumes on a slide. (See, e.g., FIG. 8.) The contact angle is the angle at which the reaction volume interface meets the planar surface Wetting is the ability of the reaction volume to maintain contact with the planar surface. In general, a contact angle less than 90° (low contact angle) usually indicates that wetting of the surface is very favorable, and the reaction volume will spread over a large area of the planar surface Contact angles greater than 90° (high contact angle) usually indicates that wetting of the surface is unfavorable so the fluid will minimize contact with the surface and form a compact liquid droplet. In certain embodiments, a planar surface is used such that the contact angle of the reaction volume is less than 90°.

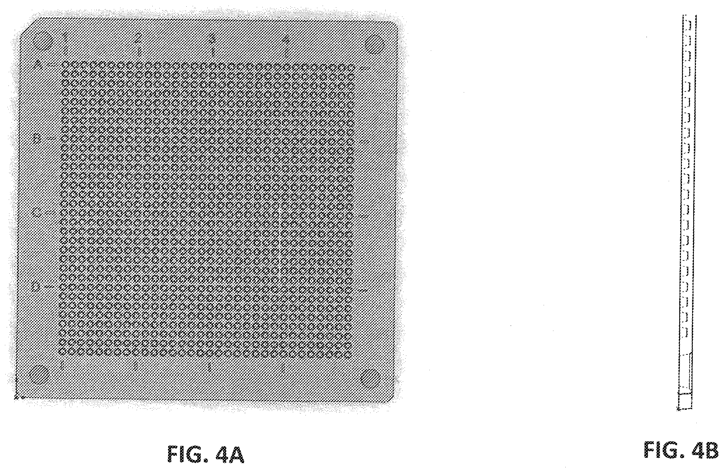

In certain embodiments, the device used in the systems and methods of the present disclosure is a microwell plate such as, for example, a nanowell array plate. FIG. 4, shows an exemplary device useful in the methods and systems of the present disclosure. As shown in FIG. 4, each nanowell has a nanoliter volume for holding a sample (e.g., nucleic acids or a cell) and the reagents necessary for a biochemical reaction. In some embodiments, the nanowell array plate is a 32 x 32 nanowell array plate. In other embodiments, the wells are 70 µm×70 µm squares with a depth of 60 µm. The nanowells can be micro fabricated using a variety of materials, including but not limited to, glass, quartz, plastics, e.g., polymethylmethacrylate (PMMA), etc., and other castable or workable polymers (e.g. polydimethylsiloxane, PDMS or SU8). Once the sample and reagent(s) have been loaded into the individual nanowells, oil or any other suitable encapsulation media is applied to isolate the discrete reaction volumes in each well.

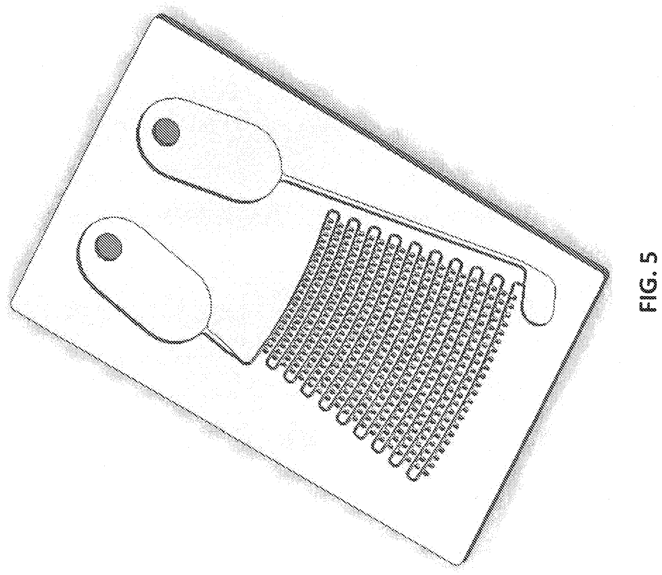

In other embodiments, the device used in the systems and methods of the present disclosure is a compact disc (CD) micro-well array. An exemplary embodiment of a CD micro-well array is shown in FIG. 5.

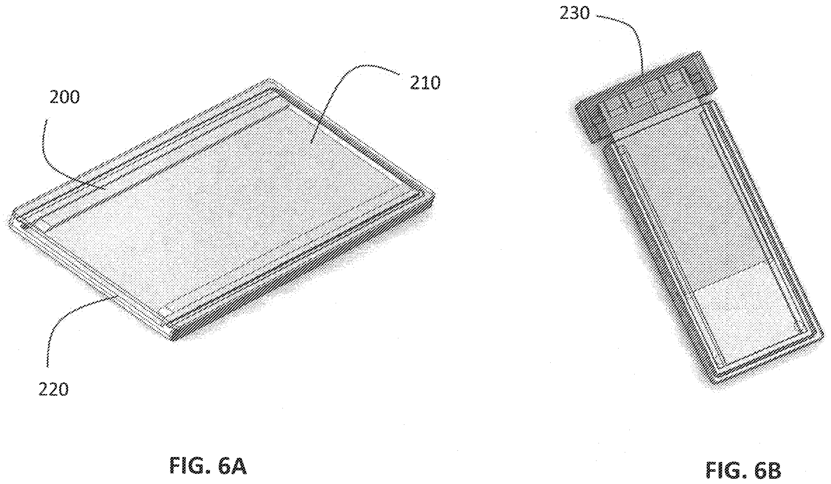

In certain aspects, the present disclosure provides apparatus for receiving a device used in the methods and systems of the present disclosure. FIG. 6A shows an exemplary apparatus (i.e., a sleeve) for receiving a slide. The apparatus comprises a base surface and a top surface 210 and sidewalls including at least one opening 220 for receiving said slide. The apparatus further comprises a pair of spaced guide rails 200 for contacting said slide to said base and preventing contact between said slide and said top surface The apparatus is configured to receive a fluid, such as, for example, oil. FIG. 63 shows a slide that has been introduced into the apparatus and a lid 230 for sealing said slide into said apparatus. For example, a slide containing a plurality of discrete reaction volumes is introduced into the apparatus shown in FIG. 6B, before or after introduction of the slide, the apparatus is filled with a fluid such as oil that is immiscible with the reaction volume.

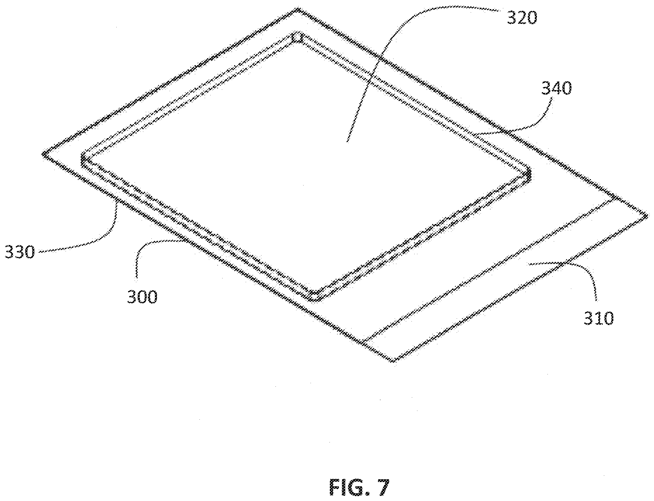

FIG. 7 shows an apparatus (i.e. a pouch) for receiving a multi-well plate used in the methods and systems of the present disclosure. As shown, pouch 300 contains a multi-well plate 340. Pouch 300 can be fabricated as a relatively thin-walled film or material. Pouch 300, can comprise one or more walls comprising a metal, a plastic, a polymer, a combination thereof, and the like. In some embodiments, pouch 70 comprises a polymer. Pouch 300 can be configured to contain a desired liquid volume, for example, from 100 uL to 10 mL, from 1 mL to 5 mL, or more. The walls of pouch 300 can be, for example, less than approximately 1.0 mm, less than 0.9 mm, less than 0.7 mm, or less than 0.5. mm in thickness, to enhance heat-transfer. Pouch 300 can have an inlet 310 through which a multi-well plate or similar device may be introduced. Pouch 300 can be sealed by heating or a glue. In some embodiments the apparatus or pouch for receiving a multi-well plate is a flexible plastic bag.

In a biochemical reaction, for example, in a PCR thermal cycling reaction, pouch 300 can be arranged and in contact with a first heating and cooling unit positioned on a first side 320, and a second heating and cooling unit comprising a heat-transfer surface in contact with side 330 of pouch 300. In some embodiments, the heating and cooling units can each comprise a Peltier unit

### Thermal Module

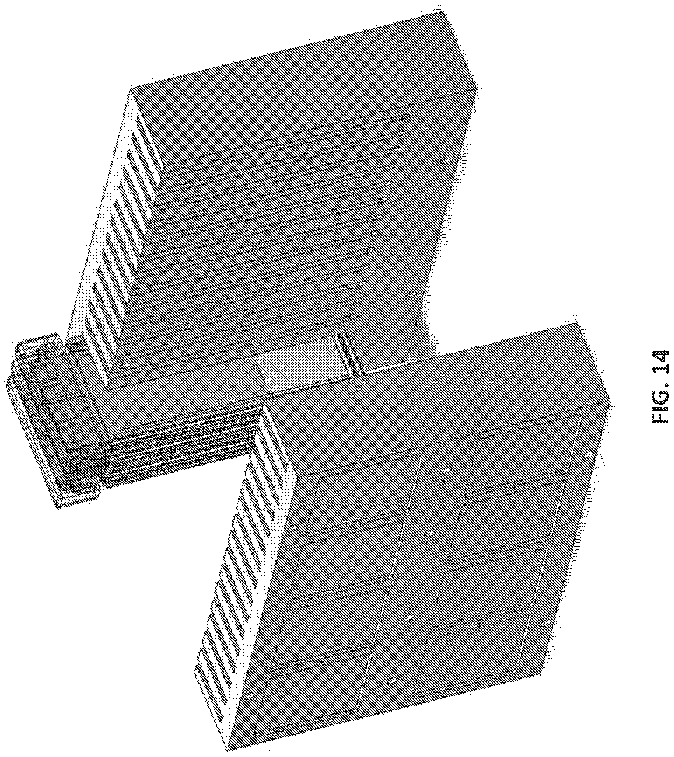

FIG. 14 illustrates an embodiment of a dual-sided thermal module useful for the systems and methods of the present disclosure. The dual-sided thermal module can comprise two or more heating elements or blocks which contact or radiate energy to opposing sides of an apparatus or device of the present disclosure. Peltier-based heating blocks similar to those used on conventional single block thermal cyclers can be adapted for use in dual-sided thermal cycling applications. Thermal cycling conducted using the two-sided heating and cooling method as described herein can provide more efficient heat-transfer in comparison to heating and cooling only one side of the apparatus or device. Single-sided heat-transfer units, such as those used by placing the apparatus or device on a conventional PCR thermal cycler with a flat block, can result in slower heat-transfer and thermal uniformity in the plurality of reaction volumes. In various embodiments, the thermal module is operably coupled to an apparatus of the present disclosure. In one aspect, the apparatus is a plastic bag containing a multi-well plate or a sleeve containing a slide. In some embodiments, the interior dimensions of the amplifier or thermal cycle are configured such that a sleeve or pouch of the present disclosure is in substantially uniform and complete contact with the heating and cooling plates.

Any effective temperature that will support the desired biochemical reaction may be employed in the isothermal biochemical reactions of this disclosure. Accordingly, the isothermal reactions may be conducted at any substantially constant and effective temperature, including at about 20° C., 21° C., 22° C., 23° C., 24° C., 25° C., 26° C., 27° C., 28° C., 29° C., 30° C., 31° C., 32° C., 33° C., 34° C., 35° C., 36° C., 37° C., 38° C., 39° C., 40° C., 41° C., 42° C., 43° C., 44° C., 45° C., 46° C., 47° C., 48° C., 49° C., 50° C., 51° C., 52° C., 53° C., 54° C., 55° C., 56° C., 33° C., 58° C., 59° C., 60° C., 61° C., 62° C., 63° C., 64° C., 65° C., 66° C., 67° C., 68° C., 69° C., 69° C., 70° C., 71° C., 33° C., 73° C., 74° C., 75° C., and the like.

In other embodiments, thermal cycling conditions are provided by a thermal module of the present disclosure. For example, in embodiments performing biochemical reactions such as DNA amplification via PCR, the temperature is cycled to produce suitable temperatures for the desired number of PCR cycles. This may be accomplished with a standard thermal cycler using a heat block or Peltier device, or it may be accomplished with alternative technologies such as an oven, hot and cold air, flowing a heated liquid with good thermal conductivity, transferring the device between instrument components held at different temperatures or any other suitable heating elements known in the art.

### Detection Module

In some embodiments, the systems and methods of the present disclosure comprise a detection module, the detection module comprising one or more optical, electrical, or chemical detectors. In certain embodiments the optical detector is a scanning detector or alternatively a stationary detector. In other embodiments, the optical detector is a photomultiplier tube, a CCD camera, photodiodes or photodiode arrays. In some aspects, the detector measures the intensity of the fluorescence signal from a discrete volume reaction.

### Recovery Module

In some embodiments, recovery modules are used to recover a portion or substantially all of an individual reaction volume for subsequent analysis and/or further biochemical reactions. Material recovery following one or more biochemical reactions may be achieved using any of the dispensing devices described herein. Material recovered from the discrete reaction volumes may optionally be used in subsequent assays or additional biochemical reactions. In some embodiments, capillary action may be used to recover a liquid in the systems and methods of the present disclosure. For example, a capillary tube can brought into contact with a target volume reaction. When the capillary tube contacts the volume reaction, the target volume reaction is recovered by capillary action without active control, such as a vacuum. Hydrophilic materials such as PMMA or glass can be used to enhance capillary action. In this aspect, the tube is transparent and thus the volume of the recovered material can be quantified. In other embodiments, recovered reaction volumes can be dispensed to a second device for subsequent assays or biochemical reactions by, for example, applying pressure on the tubing using a pipette or syringe.

In other embodiments, the pin tools described above are used to recover material following one or more biochemical reactions. (See, e.g., FIGS. 10A-B) For example, a pin is brought into contact with an individual reaction volume and an aliquot or substantially all of the reaction volume fluid adheres to the pin such that the pin can transfer the fluid to another well or surface. Pin tools can accurately recover between 2 and 200 nL of sample and/or reagents from a device used in the methods and systems of the present disclosure.

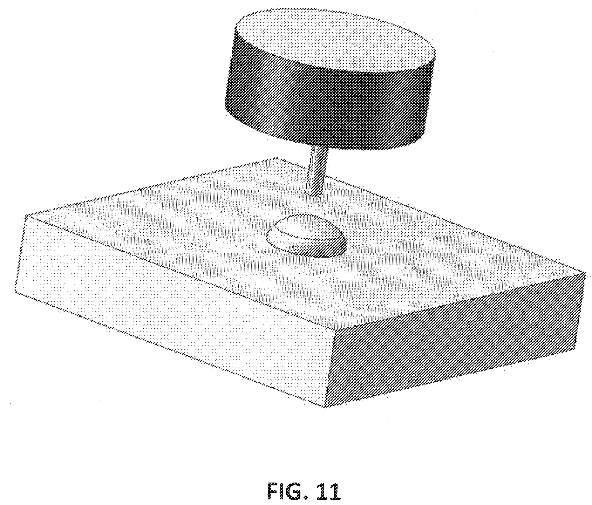

In yet other embodiments, an absorbent (e.g., porous) material is used in the systems and methods of the present disclosure to recover material following one or more biochemical reactions. FIG. 11 shows and absorbent material coming into contact with a discrete reaction volume on a planar surface of a device of the present disclosure. Any porous hydrophilic material known to one of skill in the art may be used to recover a portion or substantially all of an individual reaction volume.

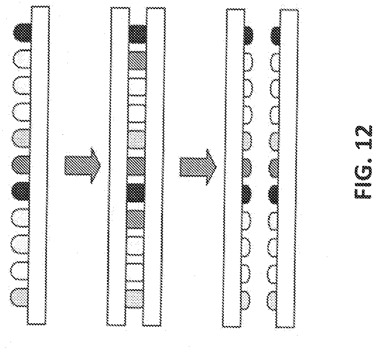

In other embodiments, recovery or splitting of a plurality of reaction volumes is achieved by bringing a first slide with a plurality of reaction volumes into contact with a second slide. As shown in FIG. 12, a plurality of discrete reaction volumes is are dispensed on a first planar surface. Next, a second planar surface is brought into contact with the discrete reaction volumes on the first planar surface. The second planar surface is removed and the original contents of the discrete reaction volumes is split into two portions. In some embodiments, additional sample and/or reagent are dispensed to individual reaction volumes after splitting.

In other embodiments, the dispensers described above are used in the systems and methods of the present disclosure to recover material following one or more biochemical reactions.

### Methods

The present disclosure provides methods for analyzing a sample, the method comprising: dispensing said sample and a reagent into a plurality of discrete reaction volumes; performing a biochemical reaction on said discrete reaction volumes; detecting a signal from said discrete reaction volumes; and recovering a portion of or substantially all reacted material from said discrete reaction volumes. In some embodiments, the discrete reaction volumes remain accessible for subsequent analysis or testing. In other embodiments, the discrete reaction volumes are not sealed.

The present disclosure further provides methods for performing serial or multiple biochemical reactions on a sample or a discrete reaction volume. As shown in Example 2 below, a plurality of discrete reaction volumes were subjected to a first whole genome amplification reaction and a second whole genome amplification. In some embodiments, the present disclosure provides methods for splitting, aliquoting, or recovering a sample to or from a device. For example, a pin tool as described above may be used to recover a portion or substantially all of a reaction volume following a biochemical reaction,

In some embodiments, the discrete reaction volumes are encapsulated by applying a hydrophobic encapsulation media to the discrete reaction volume. In some aspects, the oil is a volatile oil. Use of volatile oils in the systems and methods of the present disclosure provide access to the reaction volumes for further analysis or the introduction of additional reagents. For example, the evaporation of a volatile oil overlay can be controlled to allow for the addition of multiple reagents to reaction volumes or recovery of reaction volumes following a biochemical reaction. In some aspects, different molecular weight oils are used to provide a variety of vapor pressures, or different evaporation rates. For example, 0.65 cSt silicone oil evaporates faster than 5 cSt silicone oil. Accordingly, 0.65 cSt silicone oil can be used for low temperature incubations or high temperature incubations for a short period of time. For biochemical reactions requiring longer high temperature incubation times, 5 cSt silicone oil or high molecular weight silicone oil may be used (instead of 0.65cSt silicone oil which has a higher vapor pressure) at high temperature. Following incubation the 5cSt silicone oil or other high molecular weight oil can be removed by introducing 0.65cSt silicone oil. The 0.65cSt silicone oil is subsequently removed via evaporation following exposure to air which provides access to the reaction volumes and allows for the dispensing of additional reagents or recovery of the reaction volumes. The methods and techniques for use of one or more oils described in this methods section are applicable to the systems and apparatus described in this application.

The present disclosure provides methods for performing a biochemical reaction on a plurality of reaction volumes. In some embodiments, the disclosure further provides methods for sequencing said reaction volume. For example, following one or more biochemical reactions individual reaction volumes may be sequenced in a microreactor. Sims et al. describe techniques for fluorogenic DNA sequencing in microreactors. (Sims et at. (2011) Nature Methods 8:575-80.) In other embodiments, the disclosure further provides methods for amplifying said sample in said discrete reaction volumes. In yet other embodiments, the disclosure provides methods for performing array comparative genomic hybridization (a-CGH) on said reacted material. In other embodiments, the disclosure provides methods for genotyping said reacted material. In some embodiments, the disclosure further provides methods for measuring gene expression in said reacted material.

The systems and methods described herein can be employed in a wide variety of applications. In some embodiments, the systems and methods of the present disclosure are used to detect alleles found in a fetal genome. Currently known prenatal diagnostic methods typically involve invasive techniques such as amniocentesis, the removal chorionic villi and the removal of fetal blood or tissue biopsies. Non-invasive methods based on enriching maternal blood samples for fetal cells and analyzing the population of cells in the sample to identify fetal cells have been described. (See International Publication Nos. WO 2008/048931 and WO 2010/075459.) U.S. Patent No. 6,258,540 discloses a method of performing a prenatal diagnosis on a maternal blood sample, which method comprises obtaining a non-cellular fraction of the blood sample, amplifying a paternally inherited nucleic acid from the non-cellular fraction and performing nucleic acid analysis on the amplified nucleic acid to detect paternally inherited fetal nucleic acid. The methods described herein, in particular embodiments, may be used for the biochemical analysis of fetal genomic DNA in biological samples, such as, for example, maternal blood or plasma.

The present systems and methods can be combined with methods known in the art for determining fetal gene variants. For example, Oliphant, International Publication No. WO 2010/075459 discloses a method in which a maternal sample is genotyped, a mixture of material and fetal cells is obtained, and the sample is concentrated for fetal cells and divided into subsamples. A panel of at least one target locus at which the maternal sample is homozygous is selected for screening or genotyping of the subsamples. Each of the subsamples is individually screened or genotype at at least one of these loci, with detection of a heterozygous genotype indicating the presence of a non-maternal allele in the subsample. Alternatively, a panel of at least one target locus at which the maternal sample is heterozygous is selected for screening or genotyping of the subsamples. Each of the subsamples is individually screened or genotyped at at least one of these loci, with detection of a homozygous genotype indicating the presence of a non-maternal allele in the subsample. The systems and methods for biochemical analysis described herein are particularly suited for use in that and other methods described in Oliphant, and provide a way of detecting and analyzing fetal DNA in maternal samples, particularly by amplification and detection of fetal genomic material.

### Example 1: Genomic Analysis of Nucleic Acid Samples

A biological sample was analyzed for the presence of a rare allele (e.g., a fetal allele). Twenty microliters of an admixture of maternal and fetal DNA (0.5 ng/uL) was mixed with 35 uL of 2X Genotyping Master Mix (Part No. 4371353, Life Technologies, Carlsbad, CA), 1.75 uL of TaqMan SNP genotyping pre-mix (40X, dbSNP:rs4144457) and 13.25 uL of DEPC H2O. The resulting volume was dispensed with a dispenser (Biodot, Irvine, CA) onto a COP slide (Zeon Chemicals, Louisville, KY) in a 20x40 array to form 800 discrete reaction volumes of 30 nL. After dispensing, the slide was inserted into a plastic sleeve of the present disclosure containing 1 mL of FC40 oil (3M, St. Paul, MN). A plastic cap was used to enclose the slide in the sleeve. The oil encapsulation prevents reaction volume evaporation and cross-contamination. Guide rails in the sleeve prevented contact of the discrete reaction volumes with the top surface of the sleeve. The sleeve was then inserted into a thermal cycler and subjected to the following heating cycles: 1 cycle of 50°C for 2 minutes, 1 cycle of 95°C for 10 minutes, and 46 cycles of 95°C for 15 seconds and 60°C for 1 minute. Following thermal cycling, the slide was removed from the sleeve and exposed to air to evaporate the oil, and placed in an imaging module to detect fluorescent signals (VIC and FAM) from the discrete reaction volumes contained on the surface of the slide.

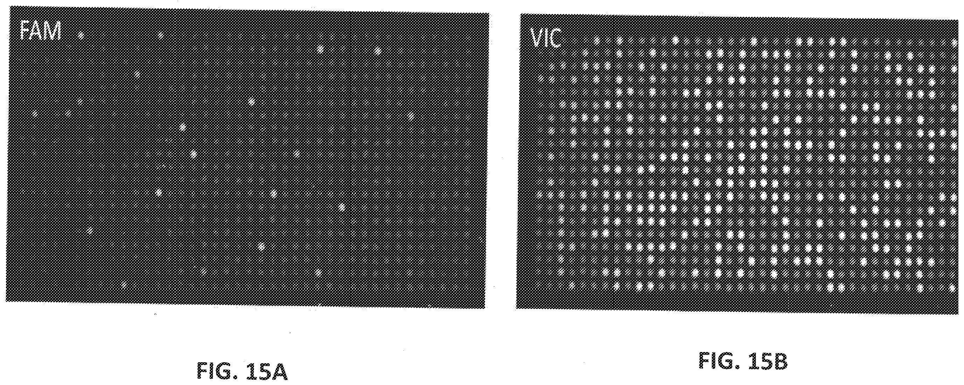

The results are shown in FIGS. 15A-B. FIG. 15A shows reaction volumes positive for the rare allele. FIG. 15B shows reaction volumes positive for the major allele. The FAM/VIC positive ratio was 7.9%. These results showed that the systems and methods in embodiments of the present disclosure are useful for performing genetic analysis on samples containing nucleic acids. These results also showed that the systems and methods in embodiments of the present disclosure are useful for performing quantitative and highly parallel biochemical reactions on biological samples in a high throughout manner.

### Example 2: Serial Biochemical Reactions on Cells and Subsequent Genomic Analysis

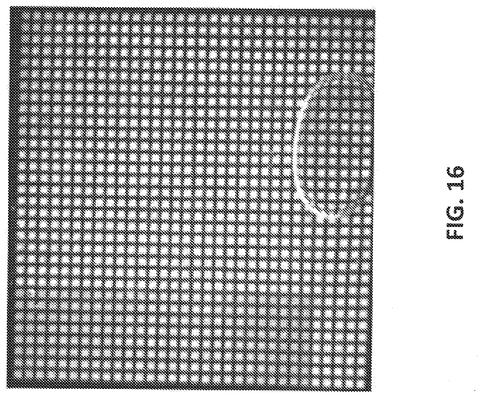

Fresh human cells (9802 cells) were resuspended in PBS (0.1 cell/nL) and dispensed in 10 nL aliquots into each well of a 32x32 multi-well plate using a dispenser (Biodot, Irvine, CA) to deliver, on average, a single cell to each well in the plate to form discrete reaction volumes. Next, 20 nL of a lysis buffer (200mM KOH and 83mM DTT) was dispensed into each well. The plate was inserted into a pouch of the present disclosure containing 5.0 cSt silicone oil, thereby encapsulating the reaction volume in each well. The oil encapsulation prevents reaction volume evaporation and cross-contamination. The pouch was heat sealed and the reaction volumes were subjected to a first biochemical reaction (i.e., cell lysis) at 65°C for 10 minutes. Following incubation, the plate was removed from the pouch and introduced into another pouch with 0.65 cSt silicone oil and gently agitated for 5 minutes to dilute the 5 cSt silicone oil present on the plate. The plate was removed from the second pouch and the 0.65 cSt silicone oil on the plate was evaporated to expose the reaction volume in each well. Next, 20 nL of neutralization buffer (900mM Tris and 200mM HCl) was dispensed into each well followed by dispensing of 150 nL of a WGA reaction mix consisting of phi29 polymerase, random primers, 2.5mM dNTPs, and a salt mix. Following the dispensing, the plate was introduced into a pouch with 0.65 cSt silicone oil, the pouch was heat sealed, and the reaction volumes were subjected to a second biochemical reaction (whole genome amplification, WGA) at 30°C for 4 hours followed by 65°C for 10 minutes to inactivate phi29 polymerase (see FIG. 16).

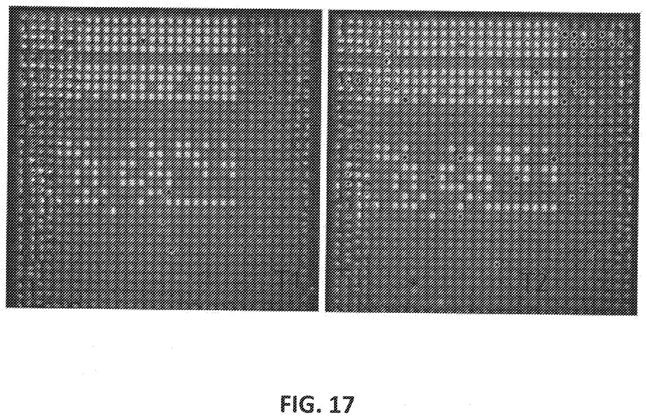

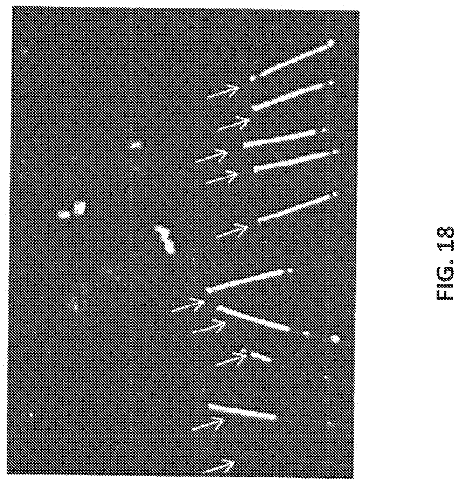

For genomic analysis, PCR was carried out on the reaction volumes following WGA. Briefly, the plate was removed from the pouch and exposed to air to remove the 0.65 silicone oil. A 32x32 pin array was introduced into the 32x32 well plate, removed, and dipped into a second 32x32 well plate that was prefilled with 150 nL of duplex PCR master mix containing primers and probes for the SRY gene (Y chromosome) and the X2 gene (X chromosome). The transfer process was repeated for a replicate plate to validate the whole genome amplification reaction. The reaction volumes in the two PCR plates were encapsulated with a 5 cSt oil overlay and incubated at 95°C for 10 minutes followed by 50 thermal cycles of 15 seconds at 95°C and 60 seconds at 60°C. Following PCR, FAM and VIC fluorescence images of the plates were taken and locations of the 9802 cells were identified by double positive signals of FAM and VIC (see FIG. 17). Samples were collected using a capillary tube-based recovery module (see FIG. 18) for further genomic analyses.

These results showed that the systems and methods in embodiments of the present disclosure are useful for performing serial biochemical reaction on cells and subsequent genomic analysis. These results also showed that the systems and methods in embodiments of the present disclosure are useful for performing quantitative and highly parallel biochemical reactions on biological samples in a high throughput manner.

## Claims

1. An integrated system for performing a biochemical reaction on a sample comprising:
a reaction module comprising: a device (30) comprising a first planar surface comprising a plurality of discrete reaction volumes, said reaction volumes comprising a reagent and a sample (10), wherein said reaction volume is accessible to perform more than one biochemical reaction on said sample;
a dispensing module (20) for dispensing said sample onto the device, wherein said dispensing module is operably coupled with said device;
a thermal module (40) comprising a heating element and a thermal control element, wherein said thermal module is operably coupled to said device;
a detection module (50) to detect a signal from the discrete reaction volumes;
a recovery module (60) comprising a second planar surface operable to individually recover at least a portion of reacted material from each of said plurality of discrete reaction volumes by bringing said second planar surface into contact with said plurality of discrete reaction volumes on the first planar surface such that, when the second planar surface is removed, said plurality of discrete reaction volumes is split into two pluralities of discrete reaction volumes disposed on the first and second planar surfaces respectively.

2. The system of claim 1, wherein said device is a slide, a micro-well plate, or a compact-disc micro-well array.

3. The system of claim 1, wherein the volume of each said reaction volume is sufficiently small to enhance reaction kinetics compared to a bulk reaction.

4. The system of claim 1, wherein said reaction volume is less than 1 microliter.

5. The system of claim 3 , wherein said sample is a cell, a nucleic acid, a whole genome, a crude cell lysate, a buccal swab, plasma, serum, whole blood, or urine.

6. The system of claim 1, wherein said reagent is a lysis buffer, a neutralization buffer, or an amplification mix.

7. The system of claim 1, wherein said reaction volume is contained in a reaction chamber, said reaction chamber configured to receive said reagent and said sample.

8. The system of claim 1, wherein said reagent is sufficient to perform a polymerase chain reaction or isothermal amplification.

9. The system of claim 3, wherein the system further comprises a sequencer for sequencing said reacted material.

10. The system of claim 1, wherein said recovery module comprises a pin tool, a pipette, a slide, or an absorbent material.

11. The system of claim 1, wherein said heating element is a dual-heating element.

## Patentansprüche

1. Integriertes System zum Durchführen einer biochemischen Reaktion an einer Probe, umfassend:
ein Reaktionsmodul, umfassend: eine Vorrichtung (30), umfassend eine erste planare Fläche, umfassend eine Mehrzahl diskreter Reaktionsvolumina, wobei die Reaktionsvolumina ein Reagens und eine Probe (10) umfassen, wobei das Reaktionsvolumen zugänglich ist, um mehr als eine biochemische Reaktion an der Probe durchzuführen;
ein Abgabemodul (20) zum Abgeben der Probe auf die Vorrichtung, wobei das Abgabemodul betriebsfähig mit der Vorrichtung gekoppelt ist;
ein thermisches Modul (40), umfassend ein Heizelement und ein thermisches Steuer-/Regelelement, wobei das thermische Modul betriebsfähig mit der Vorrichtung gekoppelt ist;
ein Erfassungsmodul (50), um ein Signal von den diskreten Reaktionsvolumina zu erfassen;
ein Rückgewinnungsmodul (60), umfassend eine zweite planare Fläche, welche betriebsfähig ist, wenigstens einen Anteil reagierten Materials jedes der Mehrzahl von diskreten Reaktionsvolumina individuell rückzugewinnen, indem die zweite planare Fläche in Kontakt mit der Mehrzahl von diskreten Reaktionsvolumina an der ersten planaren Fläche gebracht wird, sodass, wenn die zweite planare Fläche entfernt wird, die Mehrzahl diskreter Reaktionsvolumina in zwei Mehrzahlen diskreter Reaktionsvolumina geteilt wird, welche an der ersten bzw. der zweiten planaren Fläche angeordnet sind.

2. System nach Anspruch 1, wobei die Vorrichtung ein Schieber, eine Mikrowellenplatte oder eine Compact-Disk-Micro-Well-Array ist.

3. System nach Anspruch 1, wobei das Volumen jedes des Reaktionsvolumens ausreichend klein ist, um eine Reaktionskinetik im Vergleich zu einer Bulk-Reaktion zu verbessern.

4. System nach Anspruch 1, wobei das Reaktionsvolumen kleiner als 1 Mikroliter ist.

5. System nach Anspruch 3, wobei die Probe eine Zelle, eine Nukleinsäure, ein Gesamtgenomen, ein rohes Zelllysat, ein Wangenabstrich, Plasma, Serum, Vollblut oder Urin ist.

6. System nach Anspruch 1, wobei das Reagens ein Lysepuffer, ein Neutralisierungspuffer oder eine Amplifikationsmischung ist.

7. System nach Anspruch 1, wobei das Reaktionsvolumen in einer Reaktionskammer enthalten ist, wobei die Reaktionskammer dazu eingerichtet ist, das Reagens und die Probe aufzunehmen.

8. System nach Anspruch 1, wobei das Reagens ausreichend ist, um eine Polymerase-Kettenreaktion oder eine isotherme Amplifikation durchzuführen.

9. System nach Anspruch 3, wobei das System ferner einen Sequenzer zum Sequenzieren des reagierten Materials umfasst.

10. System nach Anspruch 1, wobei das Rückgewinnungsmodul ein Stiftwerkzeug, eine Pipette, einen Schieber oder ein absorbierendes Material umfasst.

11. System nach Anspruch 1, wobei das Heizelement ein doppeltes Heizelement ist.

## Revendications

1. Système intégré pour réaliser une réaction biochimique sur un échantillon comprenant :
un module de réaction comprenant : un dispositif (30) comprenant une première surface planaire comprenant une pluralité de volumes de réaction discrets, lesdits volumes de réaction comprenant un réactif et un échantillon (10), où ledit volume de réaction est accessible pour réaliser plus d'une réaction biochimique sur ledit échantillon ;
un module de distribution (20) pour distribuer ledit échantillon sur le dispositif, où ledit module de distribution est couplé de manière fonctionnelle audit dispositif ;
un module thermique (40) comprenant un élément de chauffage et un élément de régulation thermique, où ledit module thermique est couplé de manière fonctionnelle audit dispositif ;
un module de détection (50) pour détecter un signal provenant des volumes de réaction discrets ;
un module de récupération (60) comprenant une deuxième surface planaire pouvant fonctionner pour récupérer individuellement au moins une partie du matériel ayant réagi à partir de chacun de ladite pluralité de volumes de réaction discrets en mettant ladite deuxième surface planaire en contact avec ladite pluralité de volumes de réaction discrets sur la première surface planaire de sorte que, lorsque la deuxième surface planaire est retirée, ladite pluralité de volumes de réaction discrets est divisée en deux pluralités de volumes de réaction discrets disposées sur les première et deuxième surfaces planaires respectivement.

2. Système de la revendication 1, dans lequel ledit dispositif est une lame, une plaque à micro-puits ou un réseau de micro-puits à disque compact.

3. Système de la revendication 1, dans lequel le volume de chacun desdits volumes de réaction est suffisamment petit pour améliorer la cinétique de réaction par rapport à une réaction en masse.

4. Système de la revendication 1, dans lequel ledit volume de réaction est inférieur à 1 microlitre.

5. Système de la revendication 3, dans lequel ledit échantillon est une cellule, un acide nucléique, un génome entier, un lysat cellulaire brut, un prélèvement buccal, du plasma, du sérum, du sang entier ou de l'urine.

6. Système de la revendication 1, dans lequel ledit réactif est un tampon de lyse, un tampon de neutralisation ou un mélange d'amplification.

7. Système de la revendication 1, dans lequel ledit volume de réaction est contenu dans une chambre de réaction, ladite chambre de réaction étant configurée pour recevoir ledit réactif et ledit échantillon.

8. Système de la revendication 1, dans lequel ledit réactif est suffisant pour réaliser une amplification en chaîne par polymérase ou une amplification isotherme.

9. Système de la revendication 3, dans lequel le système comprend en outre un séquenceur pour séquencer ledit matériel ayant réagi.

10. Système de la revendication 1, dans lequel ledit module de récupération comprend un outil à tige, une pipette, une lame ou un matériau absorbant.

11. Système de la revendication 1, dans lequel ledit élément de chauffage est un double élément de chauffage.
